# EUROPEAN PATENT APPLICATION

(11) **EP 4 442 281 A1**
(43) Date of publication of application: **09.10.2024**
(21) Application number: 22901414.7
(22) Date of filing: 01.12.2022
(51) Int. Cl.: A61K 47/32

(54) **NASAL VACCINE-SPRAYING FORMULATION FOR SIMULTANEOUSLY TARGETING NASAL MUCOSA AND NASOPHARYNX**

(30) Priority: 02.12.2021 JP 2021196205
(71) Applicant: Toko Yakuhin Kogyo Co., Ltd., Osaka-shi, Osaka 530-0022 (JP)
(72) Inventor: KAMISHITA, Taizou, Osaka-shi, Osaka 530-0022 (JP); MIYAZAKI, Takashi, Osaka-shi, Osaka 530-0022 (JP)
(74) Representative: Hoffmann Eitle
(86) International application number: PCT/JP2022/044399
(87) International publication number: WO 2023/100984

(57) **Abstract**

The present invention relates to a formulation base prepared by adding polyethylene glycol to crosslinked polyacrylic acid, and a formulation for spraying nasal vaccine comprising an antigen, which have optimized spray pattern targeting simultaneously both of nasal mucosa and nasopharynx.

## Description

### TECHNICAL FIELD

The present invention relates to a formulation base with optimized spray pattern targeting simultaneously both of nasal mucosa and nasopharynx, and a formulation for spraying nasal vaccine comprising an antigen.

### BACKGROUND ART

Vaccines in currently-practical use are administered by subcutaneous or intramuscular injection. Recently, vaccination through nasal administration is attracting attention as a noninvasive administration that can promote the induction of immune responses to the mucous membrane in the nasal cavity which is the forefront of systemic and protective barriers, and can avoid complicated operations and pain during administration. It has been researched and developed extensively, and some live vaccines have been put to practical use.

In nasal administration for the purpose of vaccination, ordinary nasal drops have had some problems in spray-use about adhesion and retention of sprayed formulations. As a formulation base to improve the problems, Patent Reference 1 used a gel base for spray administration comprising carboxy vinyl polymer treated by adding an outside shearing force, thereby succeeded in improving the adhesion and retention, reducing the dripping, retaining an antigen properly in the nasal mucosa, improving the usability, etc. In addition, Patent Reference 2 succeeded in obtaining an effective immune response without using an adjuvant by using this gel base with an inactivated whole influenza virion, said effective immune response can be hardly obtained through ordinary nasal administrations.

In the above-mentioned improved spray technology, however, the spray density is fine and uniform, and the sprayed formulation reaches the nasal mucosa in the front half of the nasal cavity widely and uniformly, but not much of the formulation reaches directly a deeper site, *i.e.,* the nasopharynx which is thought to be important in the immune response of vaccines. Even if the formulation does not reach the nasopharynx directly by spraying, it is expected that the formulation trapped in the nasal mucosa will flow into the inferior meatus through mucociliary clearance or simple dripping, and move to the nasopharynx, but the sprayed formulation cannot sufficiently reach pharyngeal tonsil (adenoid), eustachian tonsil, palatine tonsil, and lymph follicles in posterior pharyngeal wall, which locate in nasopharynx that forms Waldeyer's ring. This point was a problem in obtaining a higher immune response.

In addition, some spray devices for spraying to nasopharynx located in the deep nasal cavity have been studied, for example, Patent Reference 3 devised a nasal spray/spout device with a nozzle comprising a nose rest. In the device in Patent Reference 3, however, the area to be sprayed is concentrated in the deep nasopharynx, and the sprayed formulation is little showered on the nasal mucosa of anterior nasal cavity. Thus, in order to enhance the efficacy of the sprayed drug, it has been necessary to make the sprayed drug reach both of the nasal mucosa and the nasopharynx directly and simultaneously, which has been still a challenge.

### PRIOR ART

### [Patent Reference]

[Patent Reference 1] WO 2007/123193
[Patent Reference 2] WO 2014/103488
[Patent Reference 3] WO 2021/066195

### SUMMARY OF INVENTION

### (Technical Problem)

From the nostril to the nasopharynx, there is a very narrow spatial region of the upper inferior meatus in a straight line. Thus, in order to spray a formulation from a spray nozzle inserted to the nostril and then make a large amount of the formulation reach the nasopharynx (nasopharyngeal) that is a target area, it was necessary to set the spray angle extremely narrow to spray in an almost straight line. However, this spraying has a narrow spray angle, thereby the amount reaching the target area may be extremely reduced if the spray direction is slightly shifted to the nasal septum side, the nasal turbinate side, or up and down. In addition, this spraying is useful for spraying to the nasopharynx, while it is also necessary to spray to the nasal mucosa of anterior nasal cavity from the viewpoint of an effective immune response. However, this spraying in a narrow spray angle may prevent the sprayed formulation from attaching at the nasal mucosa on the way.

Thus, there has been a need for a formulation with optimized performance for a centrally dense spray pattern without having an extremely narrow angle, in which the central mist of the sprayed formulation reaches the nasopharynx and the outer periphery mist is trapped with the nasal mucosa of anterior nasal cavity, so that the sprayed formulation can reach both of the nasal mucosa and the nasopharynx simultaneously.

### (Solution to Problem)

The present inventors have extensively studied on the above problem and then have found that the spray pattern can be optimized by using a formulation base prepared by adding polyethylene glycol to crosslinked polyacrylic acid, i.e., the spray pattern is full-cone and mountain-shape distribution, thereby a formulation base for nasal vaccine can be prepared, which can target both of nasal mucosa and nasopharynx simultaneously. Based on the new findings, by evenly mixing the formulation base with an antigen, a formulation for spraying nasal vaccine can be prepared, which is expected to enhance the amount reaching the nasopharynx that is strongly involved in immune induction. The present invention may provide the following embodiments.

### (Item 1)

A formulation for spraying nasal vaccine, comprising an antigen and a formulation base prepared by adding polyethylene glycol to crosslinked polyacrylic acid.

### (Item 2)

The formulation of Item 1, wherein the polyethylene glycol is macrogol 400 and/or macrogol 4000, and the content ratio of the polyethylene glycol is 0.1 - 10.0 w/w % of the formualtion base.

### (Item 3)

The formulation of Item 1 or 2, wherein the crosslink location and crosslink density in the crosslinked polyacrylic acid are adjusted so that the carboxyl content in the crosslinked polyacrylic acid is 60.0 - 62.0 w/w %, and the crosslinked polyacrylic acid is carboxy vinyl polymer with a high crosslink density to the extent that is does not cause spinnability.

### (Item 4)

The formulation of any one of Items 1 to 3, wherein the formulation base is prepared by thickening the crosslinked polyacrylic acid with an inorganic base and/or a basic amino acid, adjusting the viscosity of the thickened one with a viscosity modulating agent and/or an outside shearing force, and then adding polyethylene glycol to the obtained one, and said formulation base has an optimized spray pattern to allow the formulation base to target both of the nasal mucosa and nasopharynx simultaneously.

### (Item 5)

The formulation of Item 4, wherein the inorganic base is sodium hydroxide, potassium hydroxide, sodium salt of a weak inorganic acid, and/or potassium salt of a weak inorganic acid,
the basic amino acid is lysine, arginine, and/or ornithine, and
the viscosity modulating agent is sodium chloride, potassium chloride, and/or calcium chloride.

### (Item 6)

The formulation of any one of Items 1 to 5, wherein the antigen is live or inactivated bacteria, virus, and/or mycoplasma.

### (Item 7)

The formulation of Item 6, wherein the virus is COVID-19, severe acute respiratory syndrome (SARS), influenza virus such as influenza type A/B, hepatitis B virus, hepatitis C virus, human immunodeficiency virus (HIV), varicella virus, measles virus, mumps virus, poliovirus, rotavirus, adenovirus, herpesvirus, human papillomavirus, or rubella virus, and
the bacteria is *Streptococcus pneumoniae, Mycobacterium tuberculosis, Bordetella pertussis, Neisseria meningitidis, Haemophilus influenzae type b, Vibrio cholerae,* or *Corynebacterium diphtheriae.*

### (Item 8)

The formulation of any one of Items 1 to 5, wherein the antigen is whole antigen, split antigen, or subunit antigen of influenza virus.

### (Item 9)

The formulation of any one of Items 1 to 5, wherein the antigen is inactivated whole COVID-19 antigen, recombinant protein antigen thereof, or various viral vectors incorporating genes encoding the antigen proteins.

### (Item 10)

The formulation of any one of Items 1 to 5, wherein the antigen is hepatitis B surface antigen (HBs antigen) and/or hepatitis B nucleocapsid antigen (HBc antigen).

### (Item 11)

A formulation base used in a formulation for spraying nasal vaccine, which is prepared by adding polyethylene glycol to crosslinked polyacrylic acid.

### (Item 12)

A rhinovaccination system of vaccine comprising a spray device in which the nozzle contacts the periphery of the external nostril to fix the direction of spraying, which is filled with the formulation of any one of Items 1 - 10.

### (Item 13)

The formulation of any one of Items 1 to 5, which is used to be intranasally administered at one-shot volume of 250 - 750 µL per nose to target both of nasal mucosa and nasopharynx simultaneously.

### (Effect of the Invention)

The formulation for spraying nasal vaccine of the present invention can target both of nasal mucosa and nasopharynx simultaneously by using a formulation base prepared by adding polyethylene glycol to crosslinked polyacrylic acid to optimize the spray pattern.

In order to further optimize the spray performance of the formulation, the use of crosslinked polyacrylic acid treated by adding an outside mechanically-shearing force makes it possible to make the drug surely reach the target areas, *i.e.,* both of nasal mucosa and nasopharynx simultaneously, which enables the drug to be attached/retained at nasal mucosa and nasopharynx for a long time and induce an effective immune response with a little amount of an antigen.

The present invention provides a formulation for spraying nasal vaccine comprising an antigen as an active ingredient, which can induce an effective immune response with a little amount of the antigen and can target both of nasal mucosa and nasopharynx simultaneously to reduce side effects of the antigen.

### BRIEF DESCRIPTION OF DRAWINGS

Fig. 1 shows a nasal spray/spout nozzle composed of a tip forming a nozzle spout and a nose rest that can touch the area around the external nostril, which shows (A) perspective view, (B) side view, and (C) top view.
Fig. 2 shows a spray device in which a syringe is attached to the nasal spray/spout nozzle shown in Fig. 1, which shows (A) perspective view, (B) side view, and (C) top view.

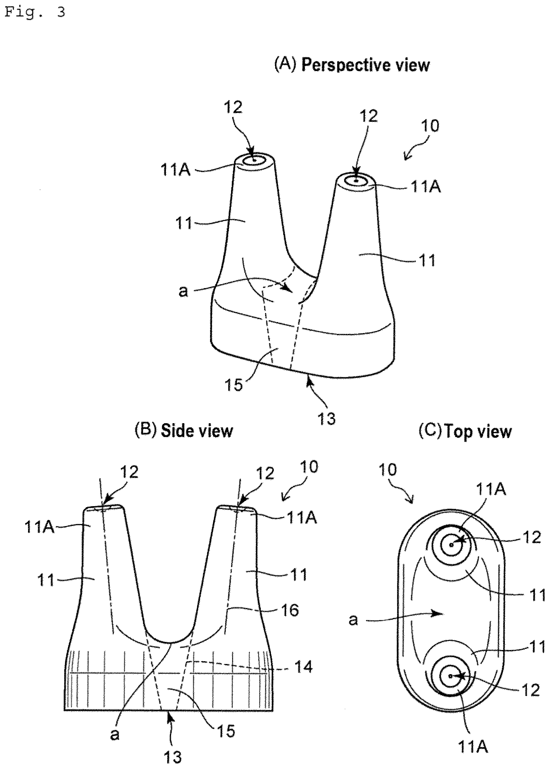

Fig. 3 shows a nasal spray/spout nozzle having a pair of sub-nozzles, in which each tip of the sub-nozzles has a nozzle spout, and the sub-nozzles are connected to each other through a connecting portion, and a separation distance between an axis of one of the sub nozzles and an axis of the other sub nozzle is increased from the connecting portion toward the tip, which shows (A) perspective view, (B) side view, and (C) top view.
Fig. 4 shows a spray device in which a syringe is attached to the nasal spray/spout nozzle shown in Fig. 3, which shows (A) perspective view, (B) side view, and (C) top view.
Fig. 5 shows the nasal cavity structure.
Fig. 6 shows a nasal cavity model used for the test for reaching target area, which includes photographs of the model from the front, side, and rear.
Fig. 7 shows the positions of nasal vestibule, nasal mucosa (former), nasal mucosa (latter), and nasopharynx in the nasal cavity model shown in Fig. 6.
Fig. 8 shows photographs of the nasal cavity model to which a test paper for trapping is placed, in the test for reaching target area.
Fig. 9 shows types of spray patterns shapes in spray pattern tests.
Fig. 10 shows actual data of the spray patterns for the test sample used in spray test 1.
Fig. 11 shows actual data of the spray patterns for the test sample used in spray test 2.
Fig. 12 shows actual data of the spray patterns for the test sample used in spray test 3.
Fig. 13 shows actual data of the spray patterns for the test sample used in spray test 4.
Fig. 14 shows actual data of the spray patterns for the test sample used in spray test 5.
Fig. 15 shows actual data of the spray patterns for the test sample used in spray test 6.
Fig. 16 shows actual data of the spray patterns for the test sample used in spray test 7.
Fig. 17 shows the results of photographed shapes and droplet size distributions in (3) "Measurement of Spray Plume Shape And Distribution of Liquid Particle Size".
Fig. 18 shows the results of HI antibody titer in serum in (5) "Test for Ability Inducing Antibody Production in Mice (2)". On the horizontal axis of the graph, formulation bases A to H are displayed in order for the four dosage volumes, in which the formulation bases comprising existing adjuvants Poly I:C and ODN2006 are designated as G and H for convenience.
Fig. 19 shows the results of IgA antibody titer in nasal lavage fluid in (5) "Test for Ability Inducing Antibody Production in Mice (2)". The explanation of the horizontal axis of the graph is the same as in Fig. 18.

### DESCRIPTION OF EMBODIMENTS

The present invention provides a formulation for spraying nasal vaccine comprising a formulation base prepared by adding polyethylene glycol to crosslinked polyacrylic acid, which has optimized the spray pattern targeting nasal mucosa and nasopharynx and delivering the drug to the both simultaneously.

The "crosslinked polyacrylic acid" used herein is a hydrophilic polymer which is prepared by polymerizing acrylic acid as the main component, wherein the crosslinking agent for making the crosslinked polymer includes, but is not limited to, for example, poly(alkenyl ether) such as allyl pentaerythritol, allyl scroll, and allyl propylene, and divinyl compound such as divinyl glycol. The cross-linked polyacrylic acid (carboxyvinyl polymer) used herein may be a polymer with a high crosslink density to the extent that it does not cause stringiness.

The "crosslink density" denotes the ratio of the number of crosslinks (structural units causing crosslinks) to the total number of structural units in a polymer that has crosslinked structures, depending on which, the polymer may have different characteristics such as fluidity, relative viscosity, and relative ion resistance. The crosslinked polyacrylic acid used herein denotes polyacrylic acid to which the properties are added by adjusting the position and density of crosslinks contained in the polymer. As the crosslink density increases, the carboxyl group content in the crosslinked polymer decreases. "The crosslink location and crosslink density in the crosslinked polyacrylic acid are adjusted so that the carboxyl content in the crosslinked polyacrylic acid is 60.0 - 62.0 w/w %, and the crosslinked polyacrylic acid is carboxy vinyl polymer with a high crosslink density to the extent that is does not cause spinnability" means that the carboxyl content is in a range of 60.0 % to 62.0 %, especially preferably in a range of 60.0 % to 61.0 %, which includes, for example, a commercial product such as Carbopol 940NF, Carbopol 941NF, Carbopol 934NF, Carbopol 980NF, Carbopol 981NF, PW-120, JUNRON PW-121, JUNRON PW-312S, AQUPEC HV-805EG, and AQUPEC HV-801EG.

The "polyethylene glycol" used herein is "macrogol", and is preferably what is distinguished as "macrogol" used for pharmaceuticals/pharmaceutical excipients. Polyethylene glycol is a high molecular compound, which has a wide molecular weight distribution, and its types are usually defined based on number average molecular weights. Polyethylene glycols having number average molecular weights of about 200 to about 20,000 are generally used. Polyethylene glycols exhibit liquid, paste, and solid properties depending on the molecular weight. In the present invention, the spray pattern is more concentrated in the central part by increasing the blending amount or molecular weight of polyethylene glycols, thus the blending amount or molecular weight of polyethylene glycols should be controlled according to the desire.

Macrogol 400 and/or macrogol 4000 are preferable for the present invention, and the blending amount is in a range of 0.1 to 10.0 % by mass, and particularly preferably, macrogol 4000 used in a range of 0.1 - 2.0 wt. %.

The "antigen" that may be included in the present invention intends to various antigens which can act as a vaccine against human, or non-human animals which includes, for example, poultry such as chicken and duck, livestock such as cattle and pigs, and pet animals such as dogs and cats. For example, it includes infectious pathogens (e.g., viruses, pathogenic bacteria, etc.), natural products purified from such pathogens, and artificial products obtained by methods such as genetic recombination such as proteins, glycoproteins, peptides, polysaccharides, lipopolysaccharides, polynucleotides, and DNA encoding an antigen. Examples of forms of antigens include virions which are complete virus particles, incomplete virus particles, virion-constituting particles, viral-nonstructural proteins, pathogen-derived proteins or glycoproteins, infection-protective antigens, and neutralizing epitopes. And, they include those with infectivity and those with lost infectivity (i.e., inactivated antigen). In addition to constitutively conventional live or inactivated vaccines, they also include component vaccines, subunit vaccines, vector vaccines, and genetic vaccines.

The viral vector used as nasal vaccine includes, for example, Sendai virus vector and human parainfluenza virus type 2 vector which are thought to have high affinity for nasal mucosa.

The antigen which is a virus includes COVID-19, severe acute respiratory syndrome (SARS), influenza virus such as influenza type A/B, hepatitis B virus, hepatitis C virus, human immunodeficiency virus (HIV), varicella virus, measles virus, mumps virus, poliovirus, rotavirus, adenovirus, herpesvirus, human papillomavirus, and rubella virus; and the antigen which is a pathogen includes *Streptococcus pneumoniae, Mycobacterium tuberculosis, Bordetella pertussis, Neisseria meningitidis, Haemophilus influenzae type b, Vibrio cholerae,* and *Corynebacterium diphtheriae.*

The "live" bacteria, virus, and/or mycoplasma used herein mean pathogenic bacteria, virus and/or mycoplasma whose toxicity is weakened to be non-pathogenic; and the "inactivated" bacteria, virus, and/or mycoplasma used herein mean pathogenic bacteria, virus and/or mycoplasma whose ability to infect is lost.

The amount of antigen contained in the vaccine formulation of the present invention varies depending on the target disease, administration subject, etc., but it is not particularly limited as long as the amount is sufficient to produce antigen-specific antibodies (IgA, IgG, etc.).

The "inorganic base" used for thickening crosslinked polyacrylic acid includes sodium hydroxide, potassium hydroxide, sodium salt of mild inorganic acid, and potassium salt of mild inorganic acid. Here, the "sodium salt of mild inorganic acid" includes disodium hydrogen phosphate hydrate, and the "potassium salt of mild inorganic acid" includes dipotassium hydrogen phosphate.

The "basic amino acid" includes lysine, arginine, and ornithine.

The "viscosity" defined herein can be measured, for example, according to Viscosity measurement by capillary tube viscometer, Viscosity measurement by rotational viscometer, etc. which are defined in the Japanese Pharmacopoeia/General Tests/Viscosity Determination/Viscosity measurement.

The "viscosity modulating agent" is an ingredient used in adjusting the viscosity of the formulation base, which is preferably selected from the group consisting of, for example, sodium chloride, potassium chloride, and calcium chloride.

The device for "adding an outside mechanically-shearing force" to adjust the viscosity to optimize the spray performance includes an ultrasonic-type stirrer, a high-speed spinning-type stirrer , a colloidal mill-type stirrer, etc. In particular, it includes, but are not be limited to, preferably a homo mixer-type, a comb-type, and an intermittently-jet-stream-generating-type high-speed spinning-type stirring devices.

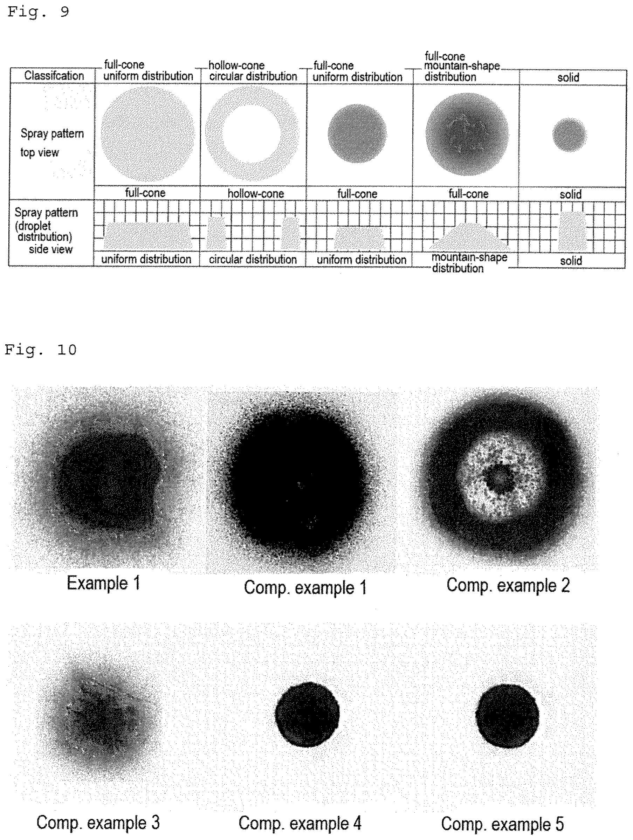

The "optimizing the spray pattern" means to control the spray pattern as full-cone and mountain-shape distribution without having an extremely narrow angle, in which the central mist of the sprayed formulation reaches the nasopharynx and the outer periphery mist is trapped with the nasal mucosa of anterior nasal cavity, so that the sprayed formulation can reach both of the nasal mucosa and the nasopharynx simultaneously. For example, it is a full-cone/mountain-shape distribution shown in Figure 9.

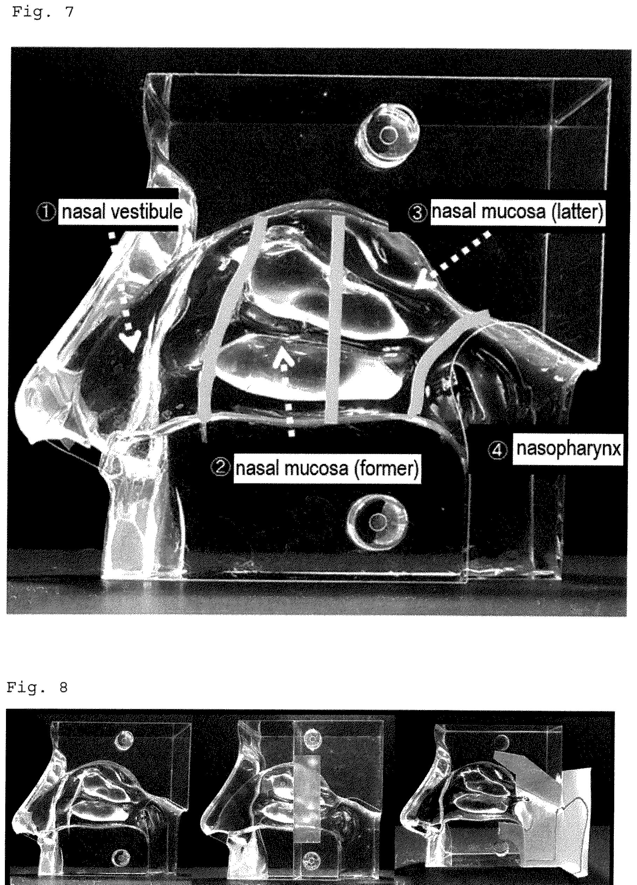

The "target area" herein denotes (1) the site centered around the nasal vestibule, which locates from the external nostril to the tip of the inferior nasal concha through the nasal valve, (2) the former half site of the nasal mucosa, which locates from the tip of the inferior nasal concha to the tip of the superior nasal concha, (3) the latter site of the nasal mucosa, which locates from the tip of the superior nasal concha to the choanae, (4) the nasopharynx (nasopharyngeal) site including eustachian tonsil and pharyngeal tonsil (see, Fig. 7).

The formulation for spraying nasal vaccine of the present invention may further comprise a pharmaceutically acceptable carrier, besides crosslinked polyacrylic acid, polyethylene glycol, and an antigen. The carrier used herein may be a carrier which is generally used in the preparation of a vaccine or a formulation for administration in nasal cavity, which includes, for example, an isotonic agent such as dextrose and glycerin, a preservative such as thimerosal, a surfactant, a stabilizing agent such as disodium edetate hydrate, and an inactivating agent such as formalin. Besides a carrier, the formulation for spraying nasal vaccine of the present inventio may comprise, for example, a suitable acidic material (phosphoric acid, citric acid, hydrochloric acid, etc.), a suitable basic material (sodium hydroxide, potassium hydroxide, basic amino acid such as lysine and arginine, etc.), various acidic buffer solution, various basic buffer solution, etc. to adjust pH to an optimum one according to the antigen.

The vaccine of the present invention is spray-administered from the nostril into the nasal cavity. For the administration of the vaccine, a multiple-dose spray container or a sprayable device to both nostrils without a pumping function, in general, a disposable sprayable device without a pumping function can be used.

The volume of the formulation for spraying nasal vaccine of the present invention to be administered into the nasal cavity is preferably 250 - 750 µL per nostril, more preferably 300 - 500 µL. In the formulation for spraying nasal vaccine of the present invention, even when the same amount of antigen is administered, increasing the volume of the formulation by a certain amount tends to enhance antibody production. Thus, the amount of the formulation to be sprayed should be determined considering the results of the examples below which were demonstrated about the tendency with mice, and the difference in the intranasal surface area between mice and humans. The above-shown numeric ranges of the volume to be administered are prefered ones under the above consideration.

The specific device for spraying the "formulation for spraying nasal vaccine" in the present invention includes "*a spray container for a gel formulation (upper-pressure-relief airless-type spray container), which is characterized in that the administration direction of the spray container can be optionally set in order to spray the preparation to an affected part for the treatment"* which is disclosed in Patent Reference 1, and "a *rhinal spray nozzle used for a medical syringe having a tip opening in fluid communication with a syringe barrel for storing a formulation"* which is disclosed in WO 2015/199130, and preferably a spray device in which the nozzle part inserted into the nose contacts the periphery of the external nostril to fix the direction of the nozzle, *i.e.,* "a spray device in which the nozzle contacts the periphery of the external nostril to fix the direction of spraying" is used. The disclosures in Patent Reference 1 and WO 2015/199130 are entirely incorporated by reference herein.

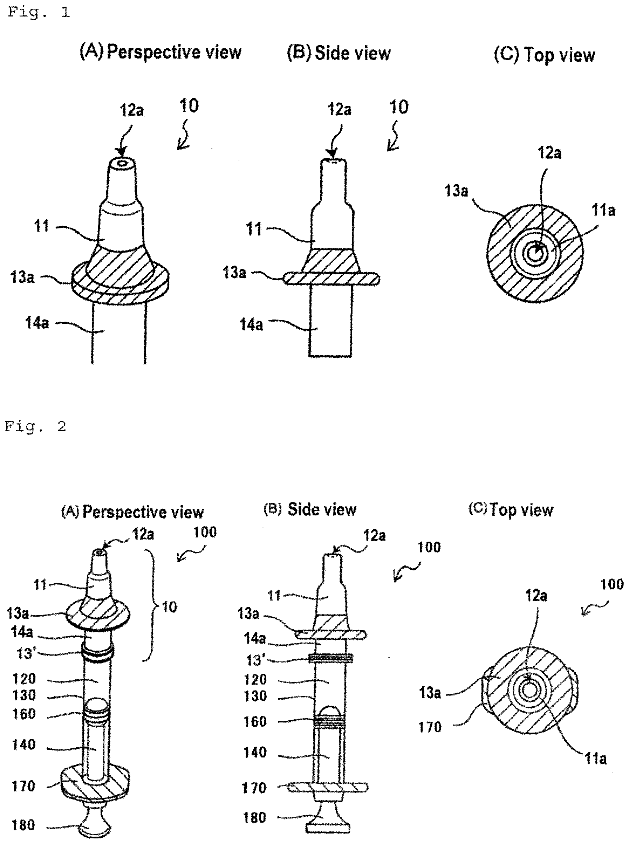

Specific examples of the "spray device in which the nozzle contacts the periphery of the external nostril to fix the direction of spraying" include a spray device equipped with a spray/spout nozzle having a nose rest, which is disclosed in Patent Reference 3 (*i.e.,* "a spray device equipped with a nasal spray/spout nozzle, which has a tip with a nozzle ejection hole formed therein and a nose rest which can contact the periphery of the external nostril"). Specifically, it is a spray device equipped with a nozzle portion having a nose rest, as shown in Fig. 1, which is attached to a syringe during use as shown in Figure 2, said device is designed so that the nose rest can come into contact with the entrance of the external nostril, fixing the entire spraying device and keeping the spray direction constant during spraying. The disclosure in Patent Reference 3 is entirely incorporated by reference herein.

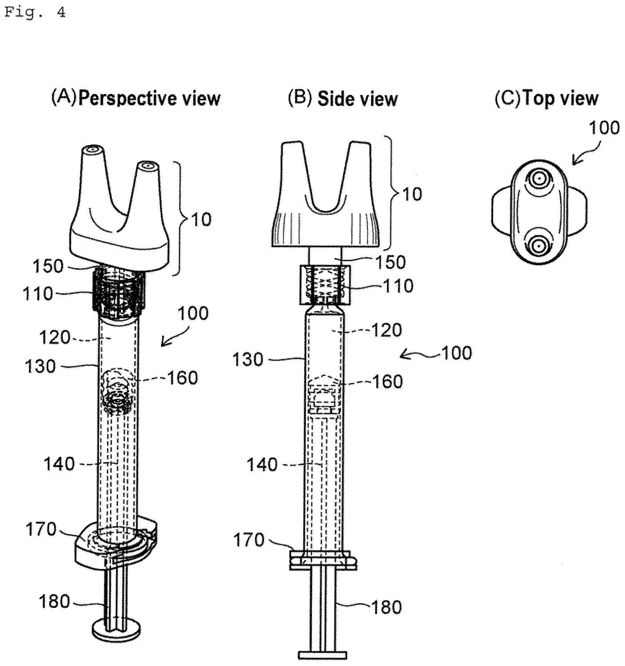

In addition, another embodiment of the "spray device in which the nozzle contacts the periphery of the external nostril to fix the direction of spraying" include a spray device equipped with a nozzle for both nostrils disclosed in Patent Reference 3, which is attached to a syringe during use as shown in Figure 4. For example, the device is a spray device equipped with a nasal spray/spout nozzle having a pair of sub-nozzles, in which each tip of the sub-nozzles has a nozzle spout, and the sub-nozzles are connected to each other through a connecting portion, and a separation distance between an axis of one of the sub nozzles and an axis of the other sub nozzle is increased from the connecting portion toward the tip.

### EXAMPLES

Hereinafter, the invention is illustrated based on some examples and some evaluations of formulations, but are not limited thereto. As for the study of formulations in the present invention, it is thought that antigen to be added does not particularly affect the spray pattern, etc., thus detailed studies of the spray characteristics were carried out using only antigen-free formulation bases.

### Example 1

Crosslinked polyacrylic acid (0.55 g) was added to purified water (74.45 g), and the mixture was stirred to become homogeneous. Then, a mechanical shearing force was given to the mixture with an intermittently-jet-stream-generating-type high-speed stirring device to adjust the viscosity. Sodium hydroxide (0.275 g), sodium chloride (0.45 g), and macrogol 4000 (1.0 g) were dissolved in purified water (25.55 g). The obtained solution was added to the above aqueous base containing crosslinked polyacrylic acid, and the mixture was stirred until homogeneous to obtain a formulation base for test as a colorless, transparent, and viscous liquid.

### Examples 2 - 8

Formulation bases of Examples 2 - 8 were prepared from the compositions shown in Table 1 in a similar means to Example 1. In addition, Example 2, in which no mechanical shearing was applied, was prepared without the step of applying mechanical shearing in Example 1.

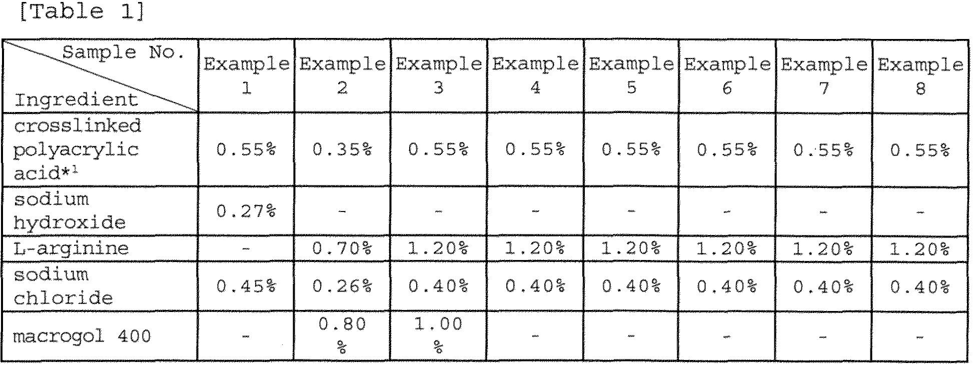

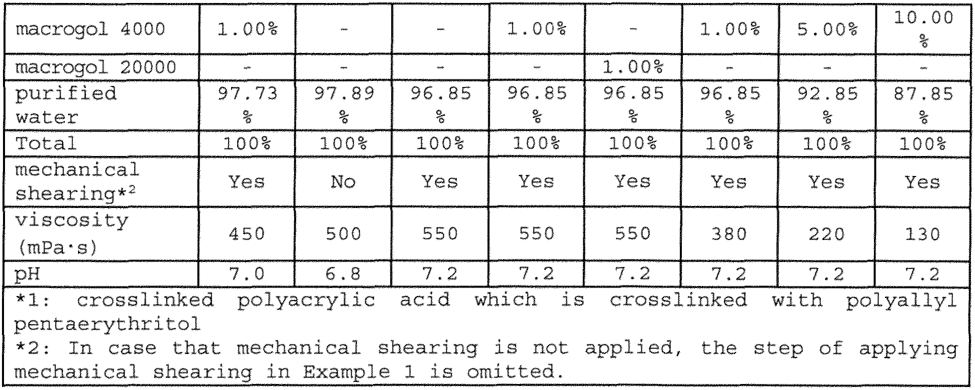

### Example 9

A formulation comprising split antigen of influenza virus as an antigen in a formulation base comprising crosslinked polyacrylic acid and polyethylene glycol was prepared in the following process.

Crosslinked polyacrylic acid (1.1 g) and concentrated glycerin (2.0 g) were added to purified water (71.9 g), and the mixture was stirred to become homogeneous. Then, a mechanical shearing force was given to the mixture with an intermittently-jet-stream-generating-type high-speed stirring device to adjust the viscosity. L-Arginine (2.4 g) and macrogol 400 (2.0 g) were dissolved in purified water (20.6 g). The obtained solution was added to the above base containing crosslinked polyacrylic acid, and the mixture was stirred until homogeneous. Then, the homogeneous mixture was sterilized under high pressure steam under appropriate conditions to give a formulation base.

To a mixture of split antigen of influenza virus (60 µg HA), sodium hydrogenphosphate hydrate (2.5 mg), potassium dihydrogen phosphate (0.4 mg), and sodium chloride (8.1 mg) was added purified water to adjust the total volume to 1.0 mL. The solution was mixed evenly to give an antigen stock solution.

The above formulation base and the above antigen stock solution were mixed in a ratio of 1:1 to give a split antigen of influenza virus formulation.

### Examples 10 - 13

Antigen formulations of Examples 10 - 13 which comprise various antigens were prepared from the compositions shown in Table 2 in a similar means to Example 9.

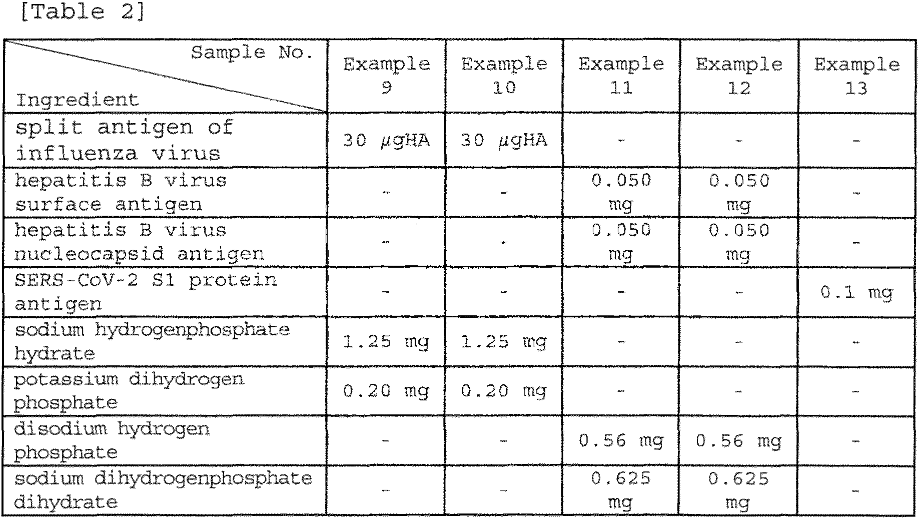

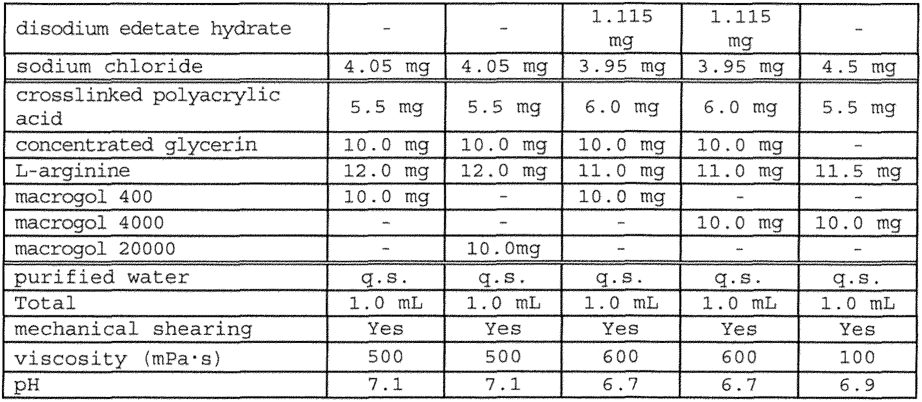

### Evaluation Test of Example Formulations

### (1) Test for Reaching Target Area

### (i) Test Mechanism and Test Method

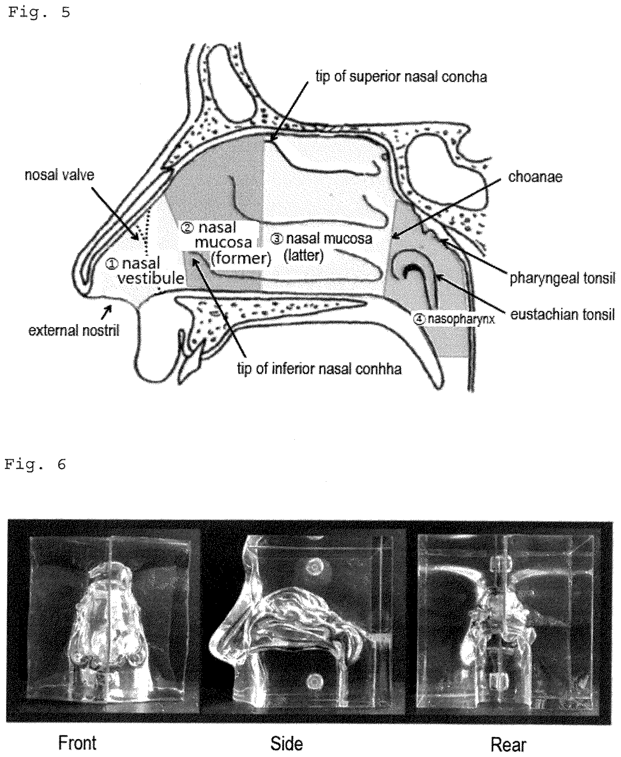

The inside of the nasal cavity has a structure as shown in Fig. 5. In order to test its reach to the target area, the spray test was conducted by using a nasal cavity model that realistically reproduces the inside of the actual human nasal cavity as complex three-dimensional structure (spray tests 1 to 7 were conducted by changing the spray formulation). The nasal cavity model herein is a nasal cavity model device made of silicone shown in Figure 6, which closely reproduces the structure of the human nasal cavity, and can be disassembled into parts as shown in Fig. 7 and Fig. 8. The amount of the formulation sprayed from the nasal cavity entrance that reaches the four sites in the nasal cavity (nasal vestibule, nasal mucosa (former), nasal mucosa (latter), and nasopharynx) is determined based on the weight that reaches each site, and the reach rate thereof is calculated.

Specifically, as for the "nasal vestibule", after spraying the formulation to the nasal cavity model, the nasal vestibule part is removed, and the reach rate is calculated based on the amount of increase in its weight. As for the deepest region "nasopharynx", a test paper is placed in the nasopharynx part, the formulation is sprayed to the nasal cavity model, and the reach rate is calculated based on the amount of increase in its weight of the test paper (see, the third photograph in Fig. 8). As for the center region of the nasal cavity, i.e., "nasal mucosa", the reach amount of the nasal mucosa is determined by subtracting the amounts that have reached the "nasal vestibule" and "nasopharynx" from the total amount of the sprayed formulation. Further, by placing a test paper in the center of the "nasal mucosa" (see, the second photograph in Fig. 8) and conducting an additional test, the amounts that have reached before and after the test paper are determined based on the amount trapped with the test paper, and the reach rates of the "nasal mucosa (former)" and the "nasal mucosa (latter)" are calculated. In the evaluation of spray tests 2 to 7 below, only the rate of reaching the "nasopharynx" was measured.

### (ii) Spray Device Used in Spray Tests

The spray tests of the present invention were conducted using any one of three types of the spray device shown in Table 3 as appropriate for the purpose of verifying the influence of the devices.

**[Table 3]**

| Device symbol | Device | Spray amount |
|---|---|---|
| Device A | an upper exhaust airless-type spray/spout device | 100 mg/shot |
| Device B | a device for nasal vaccine [1.00 mL syringe] | 250 mg/shot |
| Device C | a device for nasal vaccine [2.25 mL syringe] | 750 mg/shot |

### (iii) Influence of Spray Angle

In order to check whether the rate of reaching the target area is affected even if the spray angle is slightly off when spraying from the nasal cavity entrance, the influence on distribution to the target area is evaluated through a test in which the vertical usage angle [α] is shifted approximately 10° upwards relative to the normal insertion angle into the nose, and a test in which the horizontal usage angle [β] is shifted approximately 10° toward the nasal turbinate side. The test was conducted only in spray test 1.

### (2) Spray Pattern Test

In order to check the spray pattern of each test formulation (Examples and Comparative examples), each test formulation is put into Device B, 250 mg of the test formulation is sprayed vertically upward from the spray hole of the device at a distance of 30 mm from the test paper (a filter paper soaked in bromophenol blue, dyed, and dried), and the shape of the discolored area (yellow to blue) where the sprayed formulation is attached is observed. The types of spray patterns based on their shapes are shown in Fig. 9. The present test was conducted using all the formulations used in spray tests 1 to 7.

Hereinafter, spray tests 1 to 7 were conducted while changing the test formulations and spray devices, and the rate of reaching the target area and the spray pattern were evaluated.

### Spray test 1

In order to evaluate the effects of "crosslinked polyacrylic acid" and "polyethylene glycol" which are essential ingredients in the present invention, comparative examples (Comp. examples 1 to 5) were prepared according to the preparation method of Example 1 with the compositions shown in Table 4, in which one of the two essential ingredients is excluded from Example 1. Each sample shown in Table 4 was sprayed into the nasal cavity model device using Device B, and (1) a test for reaching target area was conducted, and additionally (2) a spray pattern of each sample was also measured.

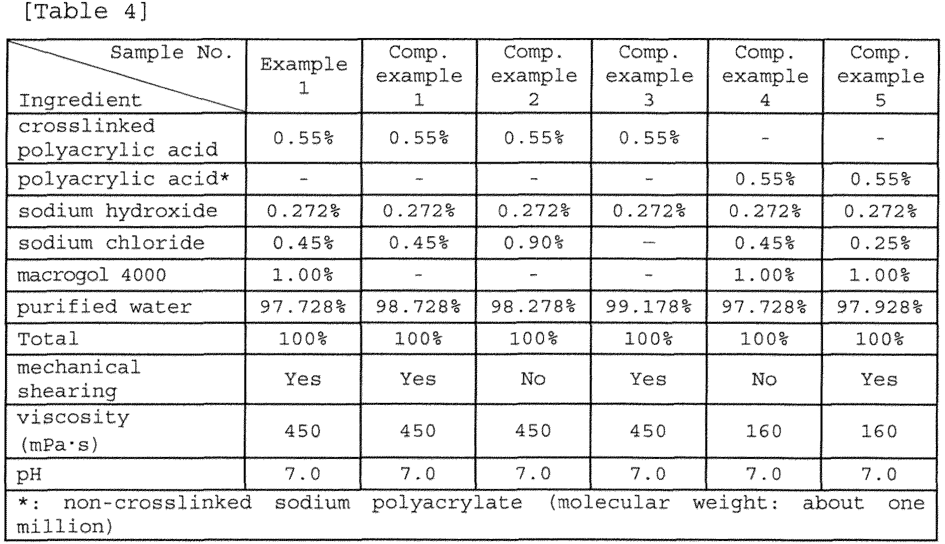

### (Result)

The results of the test for reaching target area and the spray pattern test are shown in the table below. And, the actual data of the spray pattern are shown in Fig. 10.

Example 1 which comprises crosslinked polyacrylic acid and macrogol was evenly sprayed to each target area, and additionally the spray distribution was also stable even when the spray angle was shifted. In Comparative examples 1 and 2, the amount reaching the nasopharynx was extremely small regardless of the spray angle. In Comparative examples 3 to 5, the amount reaching the nasopharynx was large, but even a slight deviation in the spray angle significantly reduced the amount reaching the nasopharynx.

**[Table 5]**

| Test sample | Test condition | | Average rate reaching target area % (n=5) | | | | Spray pattern |
|---|---|---|---|---|---|---|---|
| | device | spray angle* | nasal vestibule | nasal mucosa (former) | nasal mucosa (latter) | nasopharynx | |
| Example 1 | B | I | 6.2 % | 38.7 % | 27.6 % | 27.5 % | full-cone mountain-shape distribution |
| | | II | 7.8 % | 37.5 % | 35.6 % | 19.1 % | |
| | | III | 9.9 % | 47.9 % | 23.4 % | 18.8 % | |
| Comp. example 1 | B | I | 17.2 % | 58.5 % | 16.9 % | 7.4 % | full-cone uniform distribution (wide spray angle) |
| | | II | 19.9 % | 67.3 % | 6.5 % | 6.3 % | |
| | | III | 19.3 % | 69.5 % | 4.3 % | 6.9 % | |
| Comp. example 2 | B | I | 27.9 % | 61.7 % | 10.4 % | 0.0 % | hollow-cone circular distribution |
| | | II | 32.6 % | 64.0 % | 3.4 % | 0.0 % | |
| | | III | 30.6 % | 60.9 % | 8.5 % | 0.0 % | |
| Comp. example 3 | B | I | 0.9 % | 33.8 % | 31.2 % | 34.1 % | full-cone uniform distribution (narrow spray angle) |
| | | II | 1.5 % | 33.6 % | 53.7 % | 11.2 % | |
| | | III | 0.8 % | 40.9 % | 46.3 % | 12.0 % | |
| Comp. example 4 | B | I | 1.1 % | 11.1 % | 9.4 % | 78.4 % | solid |
| | | II | 1.7 % | 71.6 % | 22.0 % | 4.7 % | |
| | | III | 5.0 % | 64.6 % | 22.1 % | 8.3 % | |
| Comp. example 5 | B | I | 1.0 % | 12.8 % | 10.1 % | 76.1 % | solid |
| | | II | 1.4 % | 72.2 % | 25.8 % | 0.6% | |
| | | III | 2.7 % | 70.3 % | 25.8 % | 1.2 % | |
| *: Spray angle I is the standard insertion angle, Spray angle II is to be sprayed by shifting [α] approximately 10° upwards, and Spray angle III is to be sprayed by shifting [β] approximately 10° toward the nasal turbinate side. | | | | | | | |

### Spray test 2

In order to compare the effects of polyethylene glycol (macrogol) and other alcohol compounds as additives, comparative examples (Comp. examples 6 to 11) were prepared according to the preparation method of Example 1 with the compositions shown in Table 6, which comprise various alcohol compounds instead of macrogol of Example 2. Each sample shown in Table 6 was sprayed into the nasal cavity model device using Device A, and (1) a test for reaching target area was conducted, and additionally (2) a spray pattern of each sample was also measured.

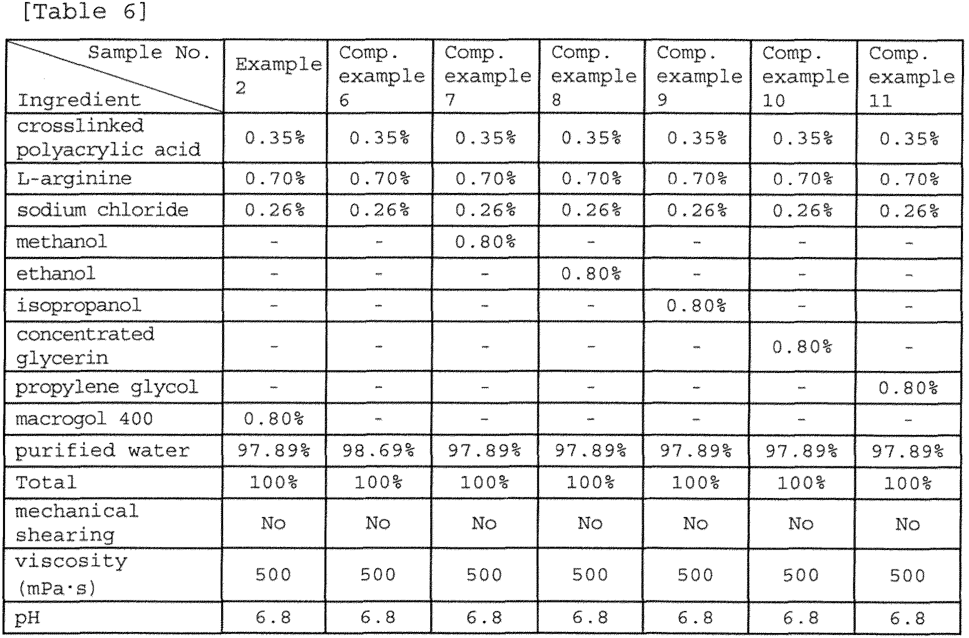

### (Result)

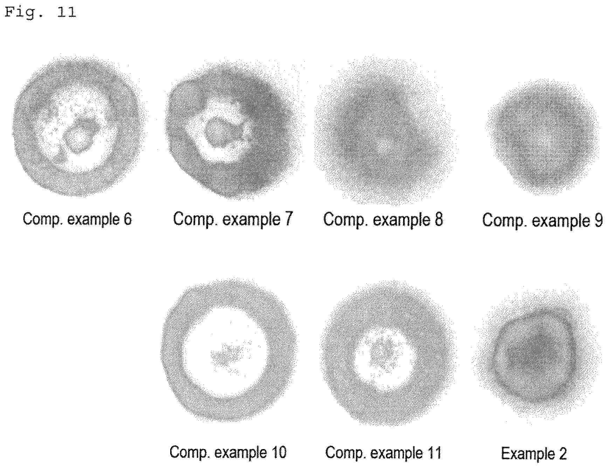

The results of the test for reaching target area and the spray pattern test are shown in Table 7. And, the actual data of the spray pattern are shown in Fig. 11.

Example 2 which comprises macrogol showed a high rate reaching nasopharynx and a good spray pattern having full-cone/mountain-shape distribution. In the comparative examples, the amount reaching the nasopharynx was small. Among them, Comparative example 9 showed relatively high reach rate, but considering the irritation and safety of isopropanol, it was predicted that it would not be suitable for administration to mucous membranes by nasal spray.

**[Table 7]**

| Test sample | Device | Average rate reaching nasopharynx % (n=5) | Spray pattern |
|---|---|---|---|
| Example 2 | A | 15.7% | full-cone/mountain-shape distribution |
| Comparative example 6 | A | 0.0% | hollow-cone/circular distribution |
| Comparative example 7 | A | 0.0% | hollow-cone/circular distribution |
| Comparative example 8 | A | 2.2% | full-cone/uniform distribution |
| Comparative example 9 | A | 8.3% | full-cone/mountain-shape distribution |
| Comparative example 10 | A | 0.0% | hollow-cone/circular distribution |
| Comparative example 11 | A | 0.1% | hollow-cone/circular distribution |

### Spray test 3

In order to evaluate the effect of differences in molecular weight of polyethylene glycol (macrogol), comparative examples (Comp. examples 12 and 13) were prepared according to the preparation method of Example 1 with the compositions shown in Table 8, which do not comprise polyethylene glycol. Each sample shown in Table 8 was sprayed into the nasal cavity model device using Device A, and (1) a test for reaching target area was conducted, and additionally (2) a spray pattern of each sample was also measured.

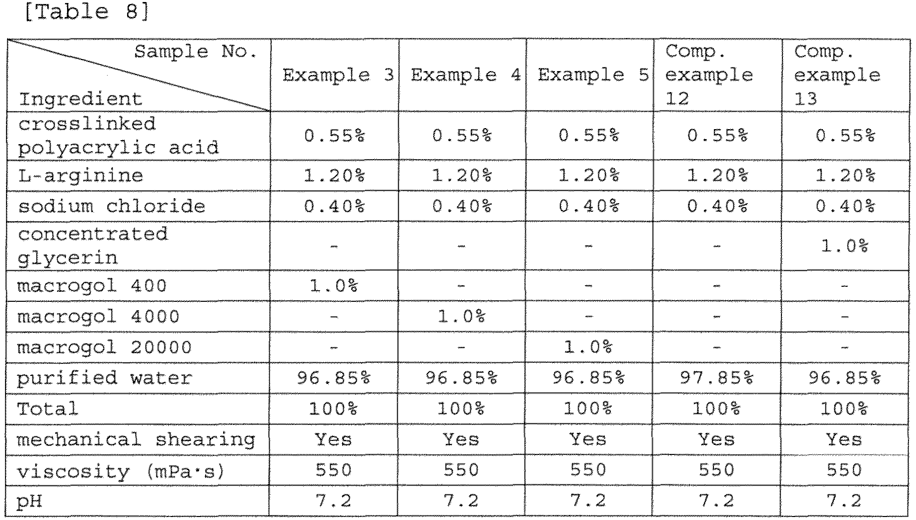

### (Result)

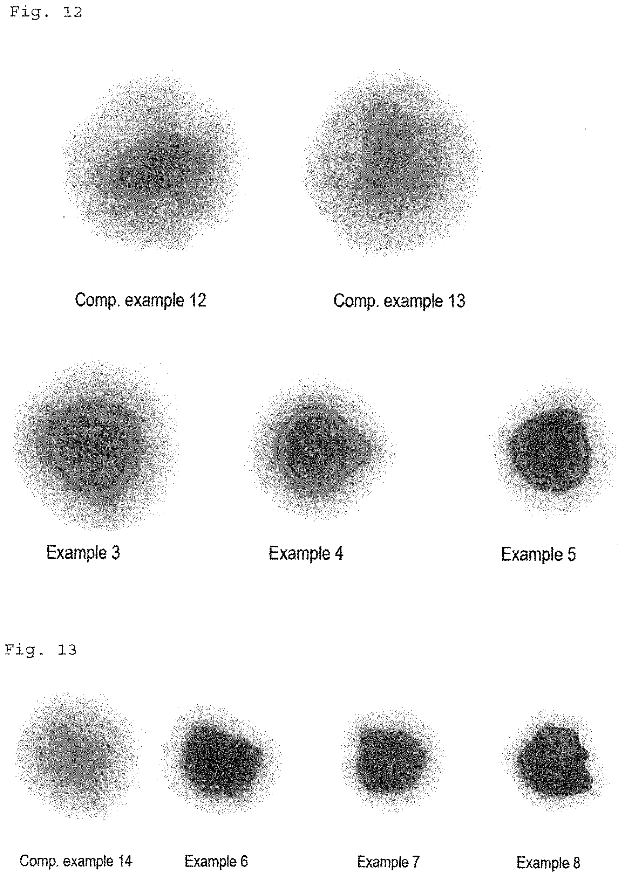

The results of the test for reaching target area and the spray pattern test are shown in Table 9. And, the actual data of the spray pattern are shown in Fig. 12.

Examples 3 - 5 which comprise macrogols with different molecular weights showed higher rate reaching nasopharynx than the comparative examples comprising no macrogol, and a good spray pattern having full-cone/mountain-shape distribution.

**[Table 9]**

| Test sample | Device | Average rate reaching nasopharynx % (n=5) | Spray pattern |
|---|---|---|---|
| Example 3 | A | 17.7% | full-cone/mountain-shape distribution |
| Example 4 | A | 20.8% | full-cone/mountain-shape distribution |
| Example 5 | A | 25.7% | full-cone/mountain-shape distribution |
| Comparative example 12 | A | 4.9% | full-cone/uniform distribution |
| Comparative example 13 | A | 6.1% | full-cone/uniform distribution |

### Spray test 4

In order to evaluate the effect of differences in concentration of polyethylene glycol (macrogol), a comparative example (Comp. example 14) was prepared according to the preparation method of Example 1 with the composition shown in Table 10, which does not comprise polyethylene glycol. Each sample shown in Table 10 was sprayed into the nasal cavity model device using Device A, and (1) a test for reaching target area was conducted, and additionally (2) a spray pattern of each sample was also measured.

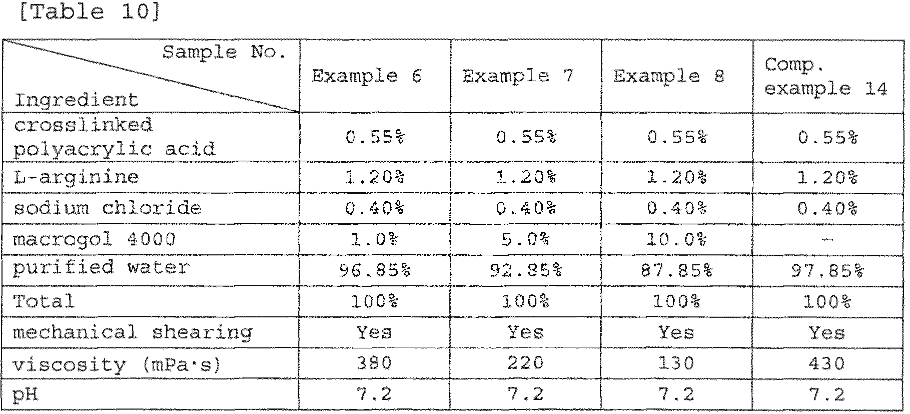

### (Result)

The results of the test for reaching target area and the spray pattern test are shown in Table 11. And, the actual data of the spray pattern are shown in Fig. 13.

Examples 6 - 8 which comprise macrogols with different concentrations showed higher rate reaching nasopharynx than the comparative example comprising no macrogol, and a good spray pattern having full-cone/mountain-shape distribution.

**[Table 11]**

| Test sample | Device | Average rate reaching nasopharynx % (n=5) | Spray pattern |
|---|---|---|---|
| Example 6 | A | 13.1% | full-cone/mountain-shape distribution |
| Example 7 | A | 14.8% | full-cone/mountain-shape distribution |
| Example 8 | A | 22.5% | full-cone/mountain-shape distribution |
| Comparative example 14 | A | 5.0% | full-cone/uniform distribution |

### Spray test 5

In order to confirm whether a formulation actually-comprising an antigen can have the same effects demonstrated in spray tests 1 to 4, Examples 9 and 10 were prepared using influenza virus split antigen as the antigen, and a comparative example (Comp. example 15) was prepared according to the preparation method of Example 9 with the composition shown in Table 12, which does not comprise polyethylene glycol (macrogol). Each sample shown in Table 12 was sprayed into the nasal cavity model device using Device B, and (1) a test for reaching target area was conducted, and additionally (2) a spray pattern of each sample was also measured.

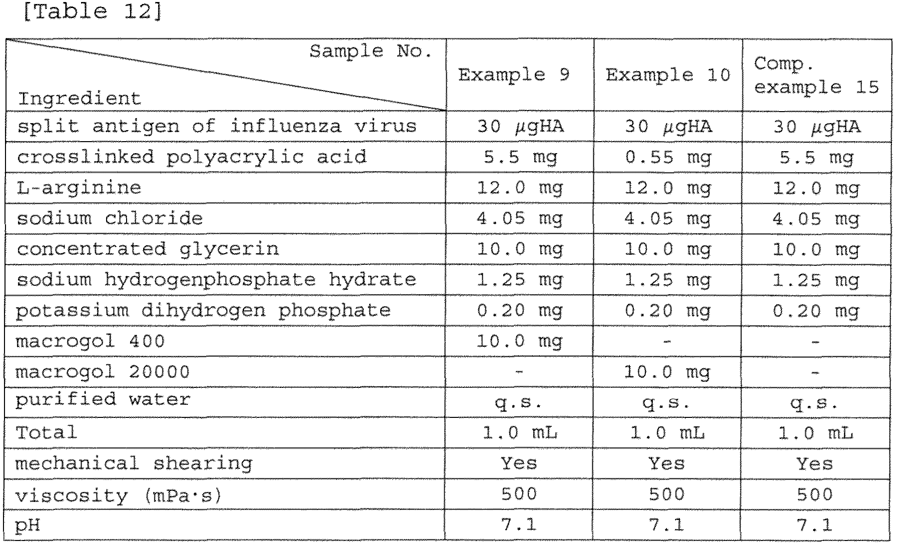

### (Result)

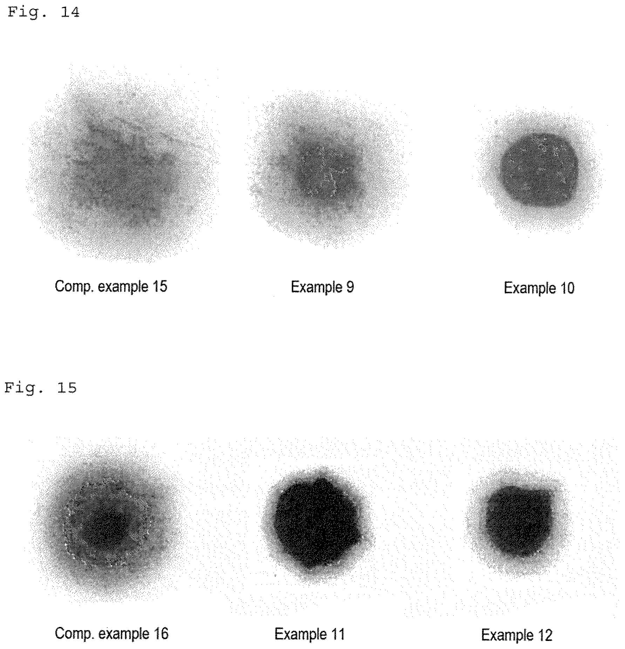

The results of the test for reaching target area and the spray pattern test are shown in Table 13. And, the actual data of the spray pattern are shown in Fig. 14.

The Examples comprising an antigen (split antigen of influenza virus) also showed higher rate reaching nasopharynx than the comparative example comprising no macrogol, and a good spray pattern having full-cone/mountain-shape distribution.

**[Table 13]**

| Test sample | Device | Average rate reaching nasopharynx % (n=5) | Spray pattern |
|---|---|---|---|
| Example 09 | B | 17.2 % | full-cone/mountain-shape distribution |
| Example 10 | B | 26.1 % | full-cone/mountain-shape distribution |
| Comparative example 15 | B | 6.4 % | full-cone/uniform distribution |

### Spray test 6

In order to confirm whether a formulation actually-comprising an antigen can have the same effects demonstrated in spray tests 1 to 4, Examples 11 and 12 were prepared using hepatitis B virus surface antigen and hepatitis B virus nucleocapsid antigen as the antigen, and a comparative example (Comp. example 16) which does not comprise polyethylene glycol (macrogol) was prepared, according to the preparation method of Example 9 with the composition shown in Table 14. Each sample shown in Table 14 was sprayed into the nasal cavity model device using Device C, and (1) a test for reaching target area was conducted, and additionally (2) a spray pattern of each sample was also measured.

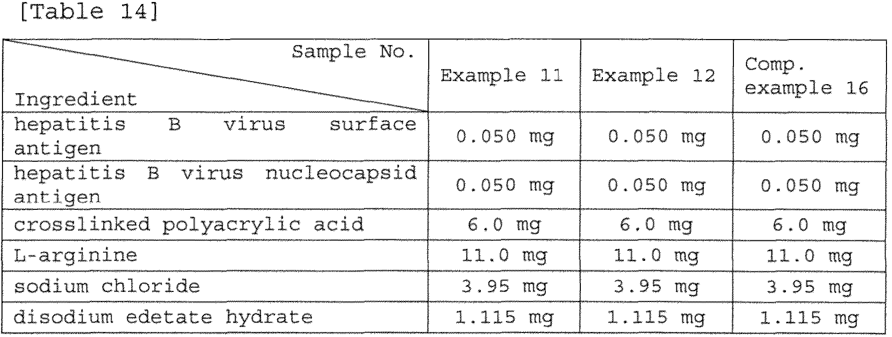

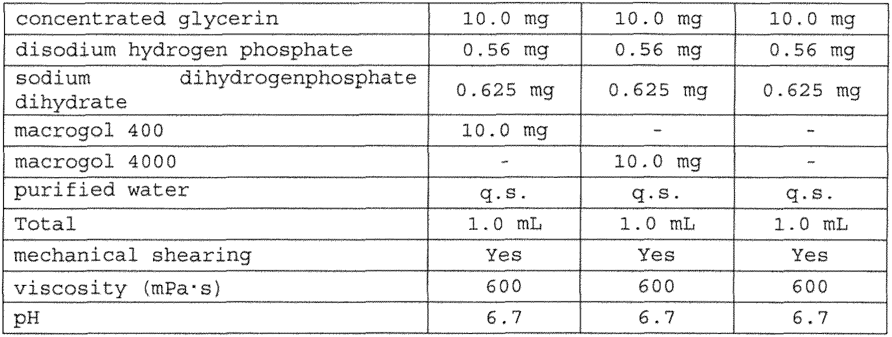

### (Result)

The results of the test for reaching target area and the spray pattern test are shown in Table 15. And, the actual data of the spray pattern are shown in Fig. 15.

The Examples comprising antigens (hepatitis B virus surface antigen and hepatitis B virus nucleocapsid antigen) also showed higher rate reaching nasopharynx than the comparative example comprising no macrogol, and a good spray pattern having full-cone/mountain-shape distribution.

**[Table 15]**

| Test sample | Device | Average rate reaching nasopharynx % (n=5) | Spray pattern |
|---|---|---|---|
| Example 11 | C | 28.0 % | full-cone/mountain-shape distribution |
| Example 12 | C | 35.6 % | full-cone/mountain-shape distribution |
| Comparative example 16 | C | 11.5 % | full-cone/uniform distribution |

### Spray test 7

In order to confirm whether a formulation actually-comprising an antigen can have the same effects demonstrated in spray tests 1 to 4, Example 13 was prepared using SARS-CoV-2 S1 protein antigen as the antigen, and comparative examples (Comp. examples 17 and 18) which do not comprise polyethylene glycol (macrogol) were prepared, according to the preparation method of Example 9 with the composition shown in Table 16. Each sample shown in Table 16 was sprayed into the nasal cavity model device using Device B, and (1) a test for reaching target area was conducted, and additionally (2) a spray pattern of each sample was also measured.

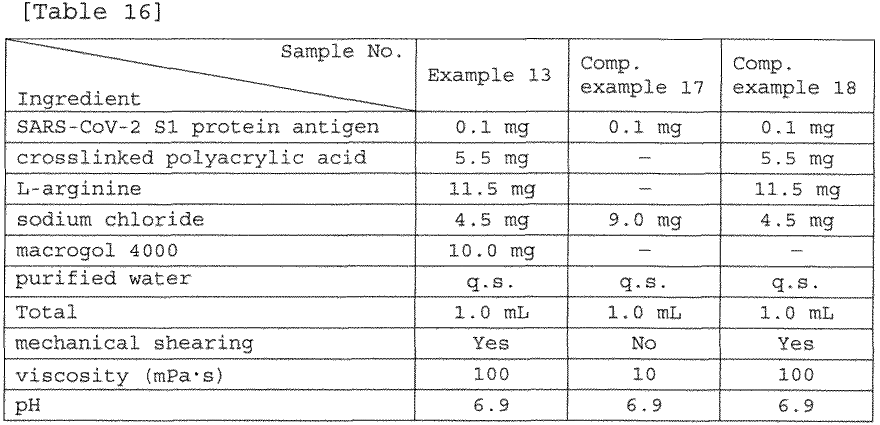

### (Result)

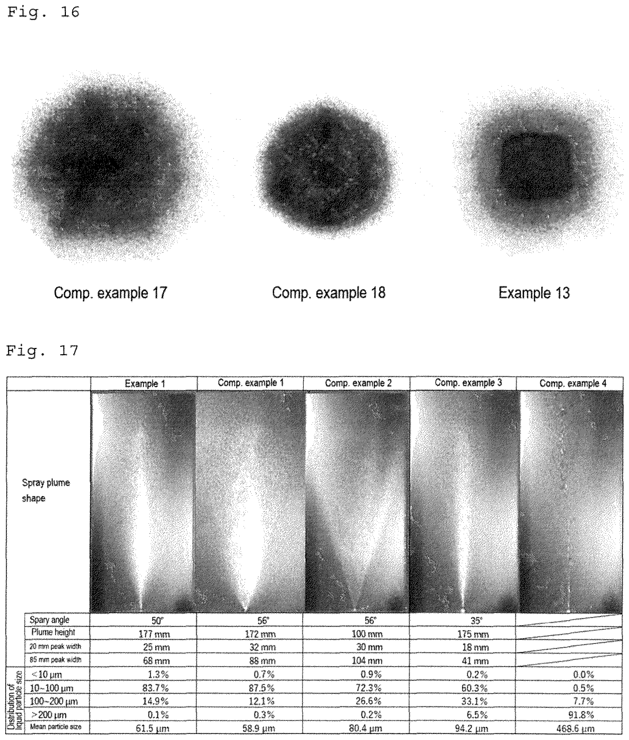

The results of the test for reaching target area and the spray pattern test are shown in Table 17. And, the actual data of the spray pattern are shown in Fig. 16.

The Example comprising an antigen (SARS-CoV-2 S1 protein antigen) also showed higher rate reaching nasopharynx than the comparative examples comprising no macrogol, and a good spray pattern having full-cone/mountain-shape distribution.

**[Table 17]**

| Test sample | Device | Average rate reaching nasopharynx % (n=5) | Spray pattern |
|---|---|---|---|
| Example 13 | B | 27.9 % | full-cone/mountain-shape distribution |
| Comparative example 17 | B | 2.0 % | full-cone/uniform distribution |
| Comparative example 18 | B | 7.1 % | full-cone/uniform distribution |

### (3) Measurement of Spray Plume Shape And Distribution of Liquid Particle Size

In order to investigate the spray plume shape and the distribution of liquid particle size, the present formulation and comparative example formulations were tested by spraying the formulations into the air.

### (Method)

The test formulation is put into Device B, and sprayed vertically upwards in an amount of 250 mg from the device. The sprayed shape is photographed from the side using a high-speed camera (High-speed Microscope VW-9000). The shape of the spray plume is observed when it reaches its maximum, and the spray angle, etc. are measured. The size distribution of the sprayed liquid particle is measured at a distance of 30.0 mm using a laser diffraction particle size analyzer (Malvern SPRAYTEC). The evaluation was conducted for Example 1 and Comparative examples 1 - 4.

The result is shown in Fig. 17.

In Example 1 comprising crosslinked polyacrylic acid and macrogol, it was observed that the shape of the spray plume was higher in density at the center than in the comparative examples. And also, regarding the distribution of liquid particle size, Example 1 showed that the average particle size was 61.5 µm and the range of 10 - 100 um was 83.7 %, resulting in a sufficiently fine spray.

### (4) Test for Ability Inducing Antibody Production in Mice (1)

The antigen-containing formulations used in spray test 7 above were administered nasally to mice, and the ability inducing antibody production was evaluated in comparison with the comparative examples.

### (Method)

The formulations of Example 13 and Comparative examples 17 and 18 used in Spray test 7 above are administered in a single dose (3 µg as SARS-CoV-2 S1 protein antigen) into both nostrils of BALB/C mice (female, 6 weeks old). Three weeks after the administration, the serum, the nasal lavage fluid, and the alveolar lavage fluid are collected, SARS-CoV-2 S1 protein-specific IgA in the nasal lavage fluid, SARS-CoV-2 S1 protein-specific IgA in the alveolar lavage fluid, and SARS-CoV-2 S1 protein-specific IgG in blood are measured to analyze the ability inducing antibody production. The administration device used herein was a microsyringe with polypropylene tube attached to the tip.

### (Result)

The result is shown in the table below.

The Example formulation showed higher antibody production at each area than the Comparative examples.

**[Table 18]**

| Test sample | SARS-CoV-2 S1 protein-specific IgA in nasal lavage fluid (Titer) | SARS-CoV-2 S1 protein specific IgA in alveolar lavage fluid (Titer) | SARS-CoV-2 S1 protein-specific IgG1 in blood (Titer) | SARS-CoV-2 S1 protein-specific IgG2a in blood (Titer) |
|---|---|---|---|---|
| No treatment | 0 | 0 | 0 | 0 |
| Comparative example 17 | 0 | 0 | 1500 | 0 |
| Comparative example 18 | 5 | 667 | 86667 | 167 |
| Example 13 | 59 | 8960 | 704000 | 44333 |

### (5) Test for Ability Inducing Antibody Production in Mice (2)

### 1. Preparation of vaccine formulation

### (1) Preparation of antigen stock solution

Influenza split antigen [H1N1] was mixed with saline until homogeneous to prepare antigen stock solutions having four concentrations shown below.

**[Table 19]**

| antigen stock solution | a | b | c | d |
|---|---|---|---|---|
| concentration | 2.0 pgHA/5 µL | 2.0 µgHA/10 µL | 2.0 pgHA/15 µL | 2.0 µgHA/30 µL |

### (2) Preparation of formulation base

The ingredients shown in the table below were mixed in purified water, neutralized to around pH 7 with sodium hydroxide, and mixed until homogeneous to prepare each formulation base. The composition treated "by adding an outside shearing force" was prepared by giving a mechanical shearing force to the composition with an intermittently-jet-stream-generating-type high-speed stirring device (formulation bases A - C correspond to the formulation bases of Examples of the present invention, and formulation bases D - E correspond to formulation bases of Comparative examples). Formulation base F was saline used as it was.

**[Table 20]**

| Ingredient | for Example | | | for Comparative example | | |
|---|---|---|---|---|---|---|
| formulation base | A | B | C | D | E | F |
| crosslinked polyacrylic acid | 11.0 mg | 11.0 mg | 11.0 mg | 11.0 mg | - | - |
| polyacrylic acid | - | - | - | - | 11.0 mg | - |
| sodium hydroxide | 5.44 mg | 5.44 mg | 5.44 mg | 5.44 mg | 5.44 mg | - |
| macrogol 400 | 20.0 mg | - | - | - | - | - |
| macrogol 4000 | - | 20.0 mg | - | - | 20.0 mg | - |
| macrogol 20000 | - | - | 20.0 mg | - | - | - |
| sodium chloride | - | - | - | - | - | 9.0 mg |
| purified water | q.s. | q.s. | q.s. | q.s. | q.s. | q.s. |
| Total | 1 mL | 1 mL | 1 mL | 1 mL | 1 mL | 1 mL |
| mechanical shearing | Yes | Yes | Yes | Yes | No | No |
| viscosity (mPa·s) | 3775 | 3900 | 4100 | 3709 | 81 | 1 |

### (3) Preparation of test sample

Test samples No. 01 - No. 24 were prepared by mixing equal amounts of an antigen stock solution and a formulation base, wherein the antigen stock solution is selected from the four types of antigen stock solutions with different concentrations and the formulation base is selected from the six types of formulation bases A to F. In addition, in order to compare the effect with the addition of an existing adjuvant, test samples No. 25 - No. 32 were prepared using formulation base F that is saline, which comprise 10 µg of an existing adjuvant (Poly I:C or ODN 2006) per the formulation to be administered into each nostril. Thus, totally 32 types of test samples shown in the table below were prepared.

**[Table 21]**

| Test sample No. | Antigen stock solution | Formulation base | Adjuvant | Viscosity after mixing (mPa·s) | Dosage volume per nose |
|---|---|---|---|---|---|
| 01 | a | A | free | 120 | 2.5 *µ*L |
| 02 | b | | | | 5.0 *µ*L |
| 03 | c | | | | 7.5 *µ*L |
| 04 | d | | | | 15.0 *µ*L |
| 05 | a | B | free | 111 | 2.5 *µ*L |
| 06 | b | | | | 5.0 *µ*L |
| 07 | c | | | | 7.5 *µ*L |
| 08 | d | | | | 15.0 *µ*L |
| 09 | a | C | free | 84 | 2.5 *µ*L |
| 10 | b | | | | 5.0 *µ*L |
| 11 | c | | | | 7.5 *µ*L |
| 12 | d | | | | 15.0 *µ*L |
| 13 | a | D | free | 143 | 2.5 *µ*L |
| 14 | b | | | | 5.0 *µ*L |
| 15 | c | | | | 7.5 *µ*L |
| 16 | d | | | | 15.0 *µ*L |
| 17 | a | E | free | 14 | 2.5 *µ*L |
| 18 | b | | | | 5.0 *µ*L |
| 19 | c | | | | 7.5 *µ*L |
| 20 | d | | | | 15.0 *µ*L |
| 21 | a | F | free | 1 | 2.5 *µ*L |
| 22 | b | | | | 5.0 *µ*L |
| 23 | c | | | | 7.5 *µ*L |
| 24 | d | | | | 15.0 *µ*L |
| 25 | a | F | Poly I:C | 1 | 2.5 *µ*L |
| 26 | b | | | | 5.0 *µ*L |
| 27 | c | | | | 7.5 *µ*L |
| 28 | d | | | | 15.0 *µ*L |
| 29 | a | F | ODN2006 | 1 | 2.5 *µ*L |
| 30 | b | | | | 5.0 *µ*L |
| 31 | c | | | | 7.5 *µ*L |
| 32 | d | | | | 15.0 *µ*L |

### (4) Test method

Using the 32 test samples listed in the table above, each sample was administered into both nostrils of BALB/C mouse (female, 8 weeks old) according to the dosage volume per nostril defined in the table above: 2.5 µL, 5.0 µL, 7.5 µL, and 15.0 µL (as for influenza split antigen, 1 µg was administered per both nostrils in all the test samples), taking special care in case that the formulation base is viscous. In order to avoid suffocation due to administration of the vaccine formulation, the formulation was administered to one nostril at least 2 minutes after administration to the other nostril. The administration device used herein was a microsyringe with polypropylene tube attached to the tip.

Three weeks after the first administration, an additional nasal administration was given in the same manner, and three weeks after the second administration, the serum and nasal lavage fluid were collected. The HI antibody titer in the serum and the antigen-specific IgA in the nasal lavage fluid were measured to analyze the ability to induce antibody production.

### (5) Test result

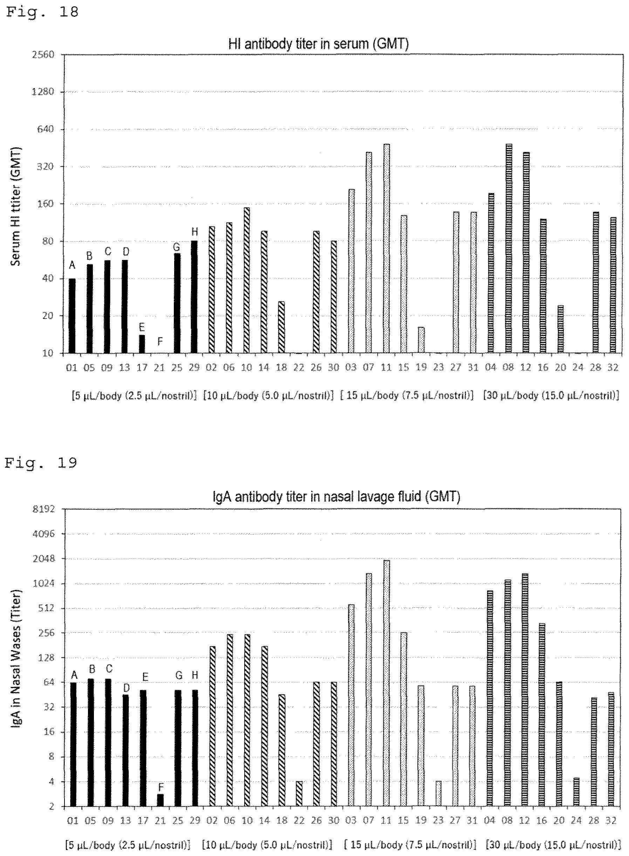

The result is shown in the table below and Fig. 18 and Fig. 19.

**[Table 22]**

| Test sample No. | Antigen stock solution | Formulation base | Adjuvant | HI antibody titer in serum | IgA antibody titer in nasal lavage fluid |
|---|---|---|---|---|---|
| 01 | a | A | free | 40 | 63 |
| 02 | b | | | 104 | 173 |
| 03 | c | | | 208 | 563 |
| 04 | d | | | 192 | 819 |
| 05 | a | B | free | 52 | 70 |
| 06 | b | | | 112 | 243 |
| 07 | c | | | 416 | 1331 |
| 08 | d | | | 480 | 1126 |
| 09 | a | C | free | 56 | 70 |
| 10 | b | | | 148 | 243 |
| 11 | c | | | 480 | 1946 |
| 12 | d | | | 416 | 1331 |
| 13 | a | D | free | 56 | 45 |
| 14 | b | | | 96 | 173 |
| 15 | c | | | 128 | 256 |
| 16 | d | | | 120 | 333 |
| 17 | a | E | free | 14 | 51 |
| 18 | b | | | 26 | 45 |
| 19 | c | | | 16 | 58 |
| 20 | d | | | 24 | 64 |
| 21 | a | F | free | 0 | 3 |
| 22 | b | | | 4 | 4 |
| 23 | c | | | 6 | 4 |
| 24 | d | | | 4 | 4 |
| 25 | a | F | Poly I:C | 64 | 51 |
| 26 | b | | | 96 | 64 |
| 27 | c | | | 136 | 58 |
| 28 | d | | | 136 | 42 |
| 29 | a | F | ODN2006 | 80 | 51 |
| 30 | b | | | 80 | 64 |
| 31 | c | | | 136 | 58 |
| 32 | d | | | 124 | 48 |

### (6) Discussion

In the present study, the amount of the antigen to be administered in each trial was adjusted to the same amount (i.e., 1 µg HA per both nostrils), but the formulation volumes to be administered were varied as 2.5 µL × 2, 5.0 µL × 2, 7.5 µL × 2, 15 µL × 2, and the production of both HI antibodies in serum and IgA antibodies in nasal lavage fluid tended to increase as increasing the formulation volume to be administered. In particular, the antibody production was greatly enhanced by increasing the volume to be administered from 5.0 µL × 2 to 7.5 µL × 2. However, the antibody production was little changed by increasing the volume to be administered from 7.5 µL × 2 to 15.0 µL × 2. These tendencies are thought to be due to the difference in the reach area of the formulation depending on the volume to be administered, i.e.,
5 µL/body (2.5 µL/nostril): the sprayed formulation may be localized to nasal cavity,
10 µL/body (5.0 pL/nostril): the sprayed formulation may reach nasal cavity and part of nasopharyngeal,
15 µL/body (7.5 pL/nostril): the sprayed formulation may reach nasal cavity, nasopharyngeal (nasopharynx), and part of oropharynx, and
30 µL/body (15.0 pL/nostril): the sprayed formulation may travel through nasal cavity and pharynx to esophagus and gastrointestinal tract.

Compared to the test samples comprising formulation base E, the test samples comprising formulation bases A to D had greater effect of enhancing the antibody production. This effect is considered to be due to the effect of crosslinked polyacrylic acid.

Compared to the test samples comprising formulation base D, the test samples comprising formulation bases A to C which comprise macrogol tended to have greater effect of enhancing the antibody production, in particular, the tendency was greater when the formulation volume is increased. And, the higher the molecular weight of macrogol, the greater the effect of enhancing antibody production. In particular, in case of the formulations comprising macrogols, the antibody production was enhanced by increasing the volume to be administered from 5.0 µL × 2 to 7.5 µL × 2.

Test samples No. 25 - No. 32 which comprise any one of two commonly-used adjuvants had clear enhancement effect of antibody production, compared with the test samples comprising formulation base F without adjuvant, but the differences in the enhancement effect due to the deviation of the formulation volume tended to be smaller compared to the test samples comprising formulation bases A to C which comprise macrogols.

### Denotation of Reference Numerals

10: a nasal spray/spout nozzle,
11a: tip portion
12a: nozzle spout
13a: nose rest
14a: nozzle body
11: sub nozzle
11A: tip portion
12: spout
13: open end
13 : protrusion for syringe connection
14: boundary line
15: connecting portion
16: axis of sub nozzle
a: crotch portion
100: syringe-type spray/spout device
110: screw tip
120: syringe barrel
130: syringe body
140: plunger rod
150: adapter
160: piston
170: finger rest
180: plunger

## Claims

1. A formulation for spraying nasal vaccine, comprising an antigen and a formulation base prepared by adding polyethylene glycol to crosslinked polyacrylic acid.

2. The formulation of claim 1, wherein the polyethylene glycol is macrogol 400 and/or macrogol 4000, and the content ratio of the polyethylene glycol is 0.1 - 10.0 w/w % of the formualtion base.

3. The formulation of claim 1 or 2, wherein the crosslink location and crosslink density in the crosslinked polyacrylic acid are adjusted so that the carboxyl content in the crosslinked polyacrylic acid is 60.0 - 62.0 w/w %, and the crosslinked polyacrylic acid is carboxy vinyl polymer with a high crosslink density to the extent that is does not cause spinnability.

4. The formulation of any one of claims 1 to 3, wherein the formulation base is prepared by thickening the crosslinked polyacrylic acid with an inorganic base and/or a basic amino acid, adjusting the viscosity of the thickened one with a viscosity modulating agent and/or an outside shearing force, and then adding polyethylene glycol to the obtained one, and said formulation base has an optimized spray pattern to allow the formulation base to target both of the nasal mucosa and nasopharynx simultaneously.

5. The formulation of claim 4, wherein the inorganic base is sodium hydroxide, potassium hydroxide, sodium salt of a weak inorganic acid, and/or potassium salt of a weak inorganic acid,
the basic amino acid is lysine, arginine, and/or ornithine, and
the viscosity modulating agent is sodium chloride, potassium chloride, and/or calcium chloride.

6. The formulation of any one of claims 1 to 5, wherein the antigen is live or inactivated bacteria, virus, and/or mycoplasma.

7. The formulation of claim 6, wherein the virus is COVID-19, severe acute respiratory syndrome (SARS), influenza virus such as influenza type A/B, hepatitis B virus, hepatitis C virus, human immunodeficiency virus (HIV), varicella virus, measles virus, mumps virus, poliovirus, rotavirus, adenovirus, herpesvirus, human papillomavirus, or rubella virus, and
the bacteria is *Streptococcus pneumoniae, Mycobacterium tuberculosis, Bordetella pertussis, Neisseria meningitidis, Haemophilus influenzae type b, Vibrio cholerae,* or *Corynebacterium diphtheriae.*

8. The formulation of any one of claims 1 to 5, wherein the antigen is whole antigen, split antigen, or subunit antigen of influenza virus.

9. The formulation of any one of claims 1 to 5, wherein the antigen is inactivated whole COVID-19 antigen, recombinant protein antigen thereof, or various viral vectors incorporating genes encoding the antigen proteins.

10. The formulation of any one of claims 1 to 5, wherein the antigen is hepatitis B surface antigen (HBs antigen) and/or hepatitis B nucleocapsid antigen (HBc antigen).

11. A formulation base used in a formulation for spraying nasal vaccine, which is prepared by adding polyethylene glycol to crosslinked polyacrylic acid.

12. A rhinovaccination system of vaccine comprising a spray device in which the nozzle contacts the periphery of the external nostril to fix the direction of spraying, which is filled with the formulation of any one of claims 1 - 10.

13. The formulation of any one of claims 1 to 5, which is used to be intranasally administered at one-shot volume of 250 - 750 µL per nose to target both of nasal mucosa and nasopharynx simultaneously.
